(19) Europäisches Patentamt
European Patent Office
Office européen des brevets

(11) EP 0 869 737 B1

(12) EUROPÄISCHE PATENTSCHRIFT

(45) Veröffentlichungstag und Bekanntmachung des Hinweises auf die Patenterteilung:
06.03.2002  Patentblatt 2002/10

(51) Int Cl.⁷: A61B 5/12

(21) Anmeldenummer: 96946211.8

(22) Anmeldetag: 19.12.1996

(86) Internationale Anmeldenummer:
PCT/DE96/02453

(87) Internationale Veröffentlichungsnummer:
WO 97/24056 (10.07.1997 Gazette 1997/30)

(54) **VERFAHREN ZUR AUTOMATISCHEN BESTIMMUNG DES HÖRVERMÖGENS, INSBESONDERE BEI NEUGEBORENEN UND KLEINKINDERN**

METHOD OF AUTOMATICALLY TESTING THE HEARING OF, IN PARTICULAR, NEW-BORN BABIES AND INFANTS

PROCEDE DE CONTROLE AUTOMATIQUE DE L'ACUITE AUDITIVE, NOTAMMENT DE NOUVEAU-NES ET DE NOURRISSONS

(84) Benannte Vertragsstaaten:
AT CH DE DK FR GB GR IT LI NL SE

(30) Priorität: 28.12.1995  DE 19548982

(43) Veröffentlichungstag der Anmeldung:
14.10.1998  Patentblatt 1998/42

(73) Patentinhaber: Pilot Blankenfelde
Medizinisch-Elektronische Geräte GmbH
15827 Blankenfelde (DE)

(72) Erfinder:
• STÜRZEBECHER, Ekkehard
D-60596 Frankfurt-am-Main (DE)
• CEBULLA, Mario
D-60320 Frankfurt am Main (DE)
• BAAG, Matthias
D-15827 Blankenfelde (DE)
• THIE, Rainer
D-15827 Blankenfelde (DE)

(74) Vertreter: Heyner, Klaus, Dr.-Ing.
Mittelweg 1h
01728 Bannewitz/Dresden (DE)

(56) Entgegenhaltungen:
US-A- 4 462 411

• MEDIZINTECHNIK, Bd. 27, Nr. 1, März 1987, BERLIN (DD), Seiten 7-9, XP000037396 A. KOPP U.A.: "Messplatz zur Ableitung akustisch evozierter Hirnstammpotentiale mit dem Elektroenzephalograph BST1"

## Beschreibung

Die Erfindung betrifft das Gebiet der objektiven, d.h. von der Mitwirkung des Patienten unabhängigen Bestimmung des Hörvermögens unter Nutzung von akustisch evozierten Potentialen (AEP) oder otoakustischer Emissionen (OAE).

Für die Anwendung der AEP entwickelte Verfahren werden vor allem bei Säuglingen und Kleinkindern angewendet, bei denen die übliche subjektive.Audiometrie (über Kopfhörer werden Töne verschiedener Frequenzen appliziert, der Patient muß angeben, ob er den jeweiligen Ton hört) nicht geeignet ist. In diesem Fall wird vorwiegend mit den frühen AEP (FAEP) gearbeitet.

Das durch einen einzelnen akustischen Reiz hervorgerufene FAEP hat eine sehr kleine Amplitude. Bei der üblichen Ableitung über Elektroden von der Kopfhaut ist es im Spontan-EEG nicht zu erkennen, das Signal-Rausch-Verhältnis ist sehr ungünstig. Zur Verbesserung des Signal-Rausch-Verhältnisses wird das sogenannte Averaging-Verfahren eingesetzt. Hierbei wird in kurzer Folge eine große Anzahl von Reizen appliziert, und die post-stimulus-Abschnitte des Spontan-EEG (Sweeps) werden summiert (gemittelt).

Unter der Voraussetzung, daß das störende Spontan-EEG Zufallscharakter hat, wird mit fortschreitender Summierung der Anteil des Spontan-EEG im Mittelungsergebnis immer geringer.

Die Mittelung muß solange durchgeführt werden, bis ein gut auswertbares AEP vorliegt. Die Entscheidung, ob ein Antwort-Potential vorliegt oder nicht, wird durch den Untersuchenden gefällt. Die angebliche objektive Audiometrie ist deshalb nur bezüglich der Datenerfassung objektiv, die Auswertung ist subjektiv.

Hier liegt bisher das wesentliche Problem der objektiven Hörschwellenbestimmung mittels der AEP: Bei Annäherung an die Hörschwelle werden die AEP stetig kleiner, das Signal-Rausch-Verhältnis wird also immer ungünstiger. Die Auswertung wird deshalb problematisch. Erschwerend kommt hinzu, daß das Spontan-EEG in aller Regel nicht, wie vorausgesetzt, ein idealer Zufallsprozeß ist. Im Mittelungsergebnis ist das schwellennahe AEP deshalb meist von einer erheblichen Reststörung überlagert. Die Auswertung erfordert deshalb viel Erfahrung. Oft hilft aber auch alle Erfahrung nicht, da im Mittelungsergebnis entweder eine zufallsbedingte Welle im AEP-Zeitbereich nicht von einem tatsächlichen AEP zu unterscheiden ist oder eine Antwort mit geringer Amplitude völlig vom Restrauschen überdeckt ist.

Eine wirksame Entscheidungshilfe ist nur von geeigneten statistischen Verfahren zu erwarten, die nicht auf das Mittelungsergebnis, sondern auf die Gesamtmenge der Einzelsweeps angewendet werden. Die Statistik kann dabei entweder auf die Zeitfunktionen der Sweeps bzw. auf aus den Zeitfunktionen abgeleitete binominale Merkmale oder, nach einer Spektral-Transformation, auf die Spektren der Sweeps angesetzt werden.
Die Entwicklung eines geeigneten Verfahrens mit statistischem (automatischem) AEP-Nachweis macht die "objektive Audiometrie" zu einer tatsächlich vollkommen objektiven Hörschwellenbestimmung.

Hierzu liegt bereits eine Reihe von Veröffentlichungen verschiedener Autoren vor, das Problem ist aber bisher noch nicht zufriedenstellend gelöst.

Als einziges praxiswirksames Ergebnis ist das US-Patent 4,275,744 von Thornton und Obenour und das nach diesem Verfahren arbeitende Gerät ALGO-1 Plus der Firma NATUS bekannt.

Das in dieser Patentschrift beschriebene Verfahren bezieht sich noch auf mittellatente AEP (MAEP), das ALGO-1 Plus arbeitet jedoch sinnvollerweise mit den für die Hörprüfung bei Säuglingen günstigeren FAEP, das Verfahren ist aber prinzipiell das gleiche:
Es wird von einem Muster-FAEP, dem sogenannten Template, ausgegangen, wie es im Mittel bei normalhörenden Kleinkindern als Antwort auf den verwendeten akustischen Click-Reiz zu erwarten ist. Das Template ergibt sich hier als Mittelwert der FAEP von 35 normalhörenden Babies bei einer Reizstärke von 35 dB HL. An Hand dieses Templates wurden 9 Datenpunkte bestimmt, die besonders stabil sind. Nur die Werte der Sweeps zu diesen Zeitpunkten werden dann bei unterschiedlicher Wichtung mittels einer Binominal-Statistik ausgewertet. Das Template ist zu den ausgewählten Zeitpunkten nach der Reizapplikation positiv bzw. negativ. Bei den einzelnen Sweeps wird nun geprüft, ob die Amplitude zu diesen Zeitpunkten ebenfalls positiv bzw. negativ ist. Die Anzahl der Übereinstimmungen wird gezählt und es wird statistisch geprüft, ob sie größer ist als bei einem reinen Zufallssignal.

Solange durch den statistischen Test kein AEP nachweisbar ist, wird weiter stimuliert, d.h. die Anzahl der einbezogenen Sweeps wird weiter erhöht, bis entweder eine Antwort nachweisbar ist oder eine vorgegebene Anzahl von Sweeps (hier 15.000) erreicht ist und die Prüfung abgebrochen wird, mit dem Ergebnis: kein AEP nachweisbar.

Entscheidender Nachteil dieses Verfahrens ist die Orientierung auf einen Muster-Potentialverlauf. Bei Vorgabe einer Musterfunktion kann nicht die relativ große interindividuelle Schwankungsbreite der FAEP-Zeitfunktionen berücksichtigt werden. Dadurch ergibt sich eine relativ große Wahrscheinlichkeit dafür, daß ein vorhandenes Potential nicht detektiert wird, weil seine Form von dem vorgegebenen Muster abweicht. Eine reine zeitliche Verschiebung des Antwortpotentials (Latenzvariation) wird allerdings durch einen Such- und Anpaßalgorithmus abgefangen, der offenbar nach jeweils 500 Sweeps neu gestartet wird.

Nachteilig ist weiterhin, daß der eingesetzte statistische Test ein sogenannter one-sample-Test ist, d.h. es werden immer nur die Bedingungen zu einem bestimmten Zeitpunkt geprüft (in diesem Fall zu den am Template markierten Zeitpunkten). Infolge der 9 geprüften Zeitpunkte liegen dann entsprechend 9 Testergebnisse vor. Die einzelnen statistischen Testergebnisse können sich widersprechen und müssen durch eine zusätzliche Prozedur zu einer Aussage verknüpft werden.

Ausgehend von dem auf das Verfahren zugeschnittenen Konzept eines Screening-Gerätes, mit dem nicht die Hörschwelle bestimmt, sondern hörgestörte Kinder "herausgesiebt" werden sollen, arbeitet das Gerät nur mit einer festen, relativ weit überschwelligen Reizintensität von 35 dB HL. Es wird als Ergebnis nur eine ja/nein-Antwort geliefert (hörgestört/nicht hörgestört).

Das Konzept ist nicht zu beanstanden, kritisch ist aber der gewählte relativ hohe Reizpegel, der auch noch bei erheblichen Hörstörungen eine nachweisbare Antwort evoziert.

Otoakustische Emissionen (OAE) sind Schallaussendungen des Innenohres, die mit einem empfindlichen Mikrofon im äußeren Gehörgang registriert werden können. OAE werden von den äußeren Haarzellen erzeugt. Die äußeren Haarzellen sind zu aktiven oszillatorischen Kontraktionen fähig, die eine sehr scharfe Frequenzabstimmung der Basilarmembran im Innenohr bewirken. Die Oszillationen der äußeren Haarzellen setzen Schwingungsenergie frei, die als retrograde Schallaussendung im äußeren Gehörgang meßbar ist. 1)

Aus dem Vorhandensein von OAE kann auf die Funktionsfähigkeit des Innenohres geschlossen werden.

Es werden verschiedene Arten von OAE unterschieden.

Die z.Zt. wichtigtsten OAE sind die

- transitorisch evozierten OAE (TEOAE) und die
- Distorsionsprodukte otoakustischer Emissionen (DPOAE).

Die OAE sind relativ einfach und mit geringem Zeitaufwand meßbar (mit kommerziellen Geräten), sie sind deshalb gut als Grundlage für einen Screening-Hörtest bei Säuglingen und Kleinkindern geeignet. Das Problem ist auch hier (wie bei den AEP), daß das Vorhandensein/Nichtvorhanden von OAE subjektiv vom Untersucher eingeschätzt werden muß. Auch hier wird wie bei den AEP das Averaging-Verfahren zur Verbesserung des Signal-Rausch-Verhältnisses eingesetzt. Es muß mit einer fest vorgegebenen Anzahl von Mittelungsschritten gearbeitet werden, die auch bei sehr kleinen OAE noch zu einem auswertbaren Ergebnis führt. Bei OAE mit größerer Amplitude wird dadurch zwangsläufig Zeit verschwendet, da sie auch bei geringerer Anzahl von Mittelungen nachweisbar wären. Für einen Einsatz als Screening-Verfahren ist aber eine möglichst kurze Untersuchungsdauer erforderlich.

Ausgehend von diesem Stand der Technik ist es Aufgabe der Erfindung, ein statistisches Verfahren mit einer höheren Potential-Nachweisempfindlichkeit anzuwenden, wodurch eine wesentlich verbesserte Bestimmung des Hörvermögens bis hin zur Hörschwellenbestimmung bei Anwendung der AEP ermöglicht werden soll und mehr diagnostische Informationen gewonnen werden können.

Eine erfindungsgemäße Lösung dieser Aufgabe ist im Patentanspruch 1 angegeben. Weiterbildungen der Erfindung sind in den Unteransprüchen gekennzeichnet.

Nach der erfindungsgemäßen Konzeption wird entweder nur im Spektralbereich (nach Anspruch 1 mit den zugehörigen Unteransprüchen) oder im Spektralbereich und im Zeitbereich (vgl. Ansprüche 11 und 12) getestet.

Da bei den FAEP das Signal-Rausch-Verhältnis im einzelnen Sweep in Schwellennähe außerordentlich ungünstig ist, erfolgt die statistische Testung nicht auf der Basis der einzelnen Sweeps, sondern es erfolgt eine Vormittelung einer ausreichenden Anzahl von Sweeps (vorzugsweise 100) zu Teilmittelwertpotentialen (TMP). Wählt man statt 100 z.B. 500 Sweeps pro Teilmittelwertpotential, ist zwar das Signal-Rausch-Verhältnis günstiger, es müssen aber, da ein minimaler Stichprobenumfang von m = 5 nicht unterschritten werden sollte, 5 • 500 = 2500 Sweeps abgeleitet werden, ehe der erste Test durchgeführt werden kann. Bereits mit 5 • 100 = 500 Sweeps, d.h. bei einem Fünftel der bei 2500 Sweeps erforderlichen Untersuchungsdauer, sind aber bei einigen Personen überschwellige FAEP nachweisbar. Bei den durchgeführten Untersuchungen hat sich die Vormittelung von jeweils 100 Sweeps als Optimum erwiesen.

Von diesen Teilmittelwertpotentialen werden die Spektren berechnet. (Mathematisch gleichwertig, aber etwas rechenaufwendiger wäre die Berechnung der Spektren aller einzelnen Sweeps und die anschließende Mittelung der Spektren.)

Die Grundlage für das erfindungsgemäße statistische Testverfahren bildet der kaum bekannte und bisher noch nicht im Zusammenhang mit dem automatischen AEP-Nachweis genannte "q-sample uniform scores Test" nach Mardia, K.V. Statistics of directional data. Academic Press, New York, 1972. Bei diesem Test werden, wie der Name sagt, q Spektralterme zugleich in die Testung einbezogen, als Ergebnis liegen nicht q Aussagen, sondern nur eine Aussage (Potential vorhanden/nicht vorhanden) vor.

Ein weiterer bisher nicht für den automatischen AEP-Nachweis verwendeter q-sample Test ist der q-sample Watson $U^2$ Test ( Maag, U.R.: A k-sample analogue of Watson's $U^2$ statistic. Biometrika 1966:53;579-583). Die Sensitivität dieses Tests ist etwas geringer als die des q-sample uniform scores Tests.

Beide q-sample Tests sind in der aus der Literatur bekannten Form nur für die Testung des Phasenspektrums

1) Sebastian Hoth, Thomas Lenarz; Otoakustische Emissionen, Grundlagen und Anwendungen, Georg Thieme Verlag Stuttgart 1993

geeignet (bei der Transformation der Zeitfunktion in den Spektralbereich erhält man ein Phasen- und ein Amplitudenspektrum). Die im Amplitudenspektrum enthaltene Information bliebe dabei ungenutzt. Der q-sample uniform scores Test wurde deshalb erfindungsgemäß so modifiziert, daß auch die Amplituden-Information in den Test eingeht. Die bereits gute Nachweisempfindlichkeit des q-sample uniform scores Tests für schwellennahe FAEP wird durch diese Modifikation noch deutlich verbessert.

Eine weitere Erhöhung der Empfindlichkeit kann ggf. dadurch erreicht werden, daß bei der schrittweisen Erhöhung der Anzahl n der getesteten Spektralterme stets eine bestimmte (ebenfalls schrittweise erhöhbare) Anzahl von Spektraltermen hinzugefügt wird, die durch Simulation erzeugt worden sind und nur zufallsverteilte Phasen und Amplituden enthalten. Enthalten die aus der realen Untersuchung stammenden Terme einen Signalanteil von einem AEP, so wird als Folge der in der Stichprobe vergrößerten Unterschiede zwischen den einbezogenen Termen der Test empfindlicher auf den Signalanteil reagieren. Enthalten die aus der realen Untersuchung stammenden Terme dagegen keinen Signalanteil von einem AEP, so sind die zugefügten Terme ohne Belang.

Die Anwendung des herkömmlichen sowie des erfindungsgemäß modifizierten q-sample uniform scores Tests auf die Spektren der Teilmittelwertpotentiale erfolgt nach einer speziellen Teststrategie, durch die eine weitere Erhöhung der Nachweis-Empfindlichkeit erzielt wird.

Es ist davon auszugehen, daß das hier ausgewertete FAEP-Spektrum aus n Spektraltermen besteht, die den für die FAEP-Darstellung wesentlichen Frequenzbereich von 30 bis etwa 600 Hz umfassen. Schwellennahe FAEP setzen sich aus vorwiegend tieferfrequenten spektralen Anteilen zusammen, mit zunehmender Überschwelligkeit des FAEP sind zunehmend auch höherfrequente Terme an der Komposition des FAEPZeitverlaufs beteiligt. Außerdem variiert die frequenzmäßige Zusammensetzung der FAEP stark interindividuell.

Der nicht modifizierte (herkömmliche) q-sample uniform scores Test prüft die Phasen der einbezogenen Spektralterme auf Gleichheit. Der modifizierte q-sample uniform scores Test bezieht zusätzlich auch die spektralen Amplituden mit ein. Da aus den beschriebenen Gründen (unbekannte spektrale Zusammensetzung des nachzuweisenden FAEP) bei einer Testsituation nicht voraussehbar ist, bei welcher Anzahl von Spektraltermen sich maximale Differenzen zwischen den Spektraltermen ergeben und damit eine optimale Nachweisempfindlichkeit erreicht wird, wurde die folgende Teststrategie entwickelt, die ein Ergebnis liefert, das sehr nahe bei der optimalen Empfindlichkeit liegt.

Nach Beginn der Potentialableitung vom Patienten werden zunächst die ersten 500 Sweeps fortlaufend zu $m = 5$ Teilmittelwertpotentialen gemittelt, und die TMP werden in den Spektralbereich transformiert.

Es kann, wie eingangs festgestellt, auch mit einer anderen Anzahl von Sweeps und einer anderen Zahl von TMP gestartet werden.

Der q-sample uniform scores Test wird dann auf diese Stichprobe, bestehend aus den 5 Spektren der TMP, mehrfach angewendet:

Beginnend z.B. mit dem der tiefsten Frequenz des Spektrums (Grundfrequenz) entsprechenden Term (vorzugsweise aber mit dem ersten Term oberhalb 50 Hz, um eine eventuelle Störung durch die Netzfrequenz auszuschließen), erfolgt ein Testlauf mit dem modifizierten q-sample uniform scores Test bei Einbeziehung einer geringen Anzahl $q_1$ von Termen. Dann wird die Anzahl auf $q_2$ erhöht, und es erfolgt ein erneuter Testlauf. Das wiederholt sich, sofern bei keinem Testlauf das Vorhandensein eines FAEP angezeigt wird, bis alle n Spektralterme einbezogen sind. Diese Mehrfachtestung kann z.B. mit $q_1=5$ oder $q_1=10$ Spektraltermen beginnen, die Erhöhung der Anzahl erfolgt dann in Schritten von z.B. 2, 5 oder 10 Spektraltermen.

Im Extremfall könnte bei ausreichend schnellem Signalprozessor die Schrittweite für die Erhöhung der Anzahl der einbezogenen Spektralterme auch 1 sein. Damit wäre das Empfindlichkeitsoptimum sogar am sichersten zu erreichen.

Die Rechenleistung moderner Signalprozessoren erlaubt eine solche Mehrfachtestung ohne zeitliche Einschränkung, da parallel zur statistischen Testung die Ableitung weiterer TMP weiterläuft.

Hat sich bei der Stichprobe mit dem Umfang von $m = 5$ auch bei Einbeziehung aller n Spektralterme keine Signifikanz ergeben (d.h. es muß bei allen durchgeführten Tests die Nullhypothese "die Ableitung enthält kein FAEP" angenommen werden), so wird bei Vorliegen der weiteren Teilmittelwertpotentiale, z.B. 5 neuer TMP, die Prozedur auf die vergrößerte Stichprobe mit dem Umfang $m = 10$ angewendet. Es erfolgt auf diese Weise eine ständige Vergrößerung des Stichprobenumfangs mit parallel laufender Testung.

Ist ein willkürlich vorgegebener maximaler Stichprobenumfang erreicht (z.B. 120, d.h. es wurden 12.000 einzelne Sweeps bei einem Reizpegel abgeleitet), ohne daß der Test Signifikanz anzeigt, wird die weitere Datengewinnung bei diesem Reizpegel abgebrochen, da der Reizpegel offenbar unterschwellig und deshalb kein FAEP vorhanden ist. Der Reizpegel kann fest eingestellt sein; er kann aber auch schrittweise erhöht oder verringert werden.

Zeigt dagegen im Verlauf der obigen Prozedur bei einem der Tests das Testergebnis an: "FAEP vorhanden", so kann das bedeuten, daß die Reizstärke noch deutlich überschwellig ist, es kann aber auch schon das an der Hörschwelle gerade noch nachweisbare Potential sein. Daraufhin wird die Testung bei diesem akustischen Reizpegel abgebrochen, oder der Pegel des Reizes wird um mindestens 5 dB verringert. Im Falle der Verringerung werden erneut die oben beschriebenen Testläufe, beginnend mit einem Stichprobenumfang von $m = 5$, gestartet.

Für die Schritte bei Verringerung bzw. Erhöhung des Reizpegels wird ein Algorithmus gewählt, der es erlaubt, die Hörschwelle bei möglichst geringem Zeitaufwand einzugrenzen.

Das Prinzip besteht darin, daß stets der Testspielraum des Reizpegels beim nächsten Test halbiert wird. Begonnen wird z.B. mit einem Reizpegel von 20 dB HL (der größte Teil der untersuchten Kinder wird normalhörend sein, deshalb ist es aus Zeitgründen sinnvoll, mit der Testung nicht sehr weit über der Normalhörschwelle zu beginnen und lieber bei den weniger häufigen schwerhörigen Kindern eine etwas längere Testzeit in Kauf zu nehmen). Zeigt der Test bei 20 dB das Vorliegen eines AEP an, wird bei diesem Reizpegel abgebrochen. Der Reizpegel wird halbiert, d. h. die nächste Testung erfolgt bei 10 dB HL. Zeigt der Test auch hier ein AEP an, wird der Reizpegel wieder halbiert auf 5 dB HL und erneut getestet. Die Untersuchung könnte aber auch abgebrochen werden, da bei einem Potential bei 10 dB HL sicher ein normales Hörvermögen vorliegt. Zeigt der Test bei 10 dB kein AEP an, wird der Reizpegel auf 15 dB erhöht. War schon bei 20 dB kein AEP nachweisbar, wird der Reizpegel auf 40 dB verdoppelt und dann bei Potentialnachweis der neue Reizpegel auf die Hälfte zwischen 40 und 20 dB = 30 dB eingestellt. Ist dagegen bei 40 dB kein Potential nachweisbar, wird der Reizpegel auf 80 dB erhöht und dann, falls hier ein Potential vorhanden ist, bei 60 dB getestet usw. Bei normalhörenden Kindern ist damit die Hörschwelle in der Regel mit 3 Schritten eingegrenzt.

Ein Problem bei jedem statistischen Test sind die sogenannten falsch-positiven Testergebnisse, d.h. der Test zeigt das Vorhandensein eines AEP an, obwohl tatsächlich kein AEP vorhanden ist, da der gewählte Reizpegel bereits unter der Hörschwelle liegt. Da die falsch-positiven Testergebnisse eine fehlerhafte Hörschwellenbestimmung zur Folge haben, muß das Auftreten solcher Testergebnisse weitestgehend verhindert werden. Für die beiden Anwendungsbereiche des Verfahrens (Hörschwellenbestimmung und Hörscreening) werden zwei unterschiedliche Möglichkeiten zur Minimierung von falsch-positiven Testergebnissen vorgeschlagen:

1. Hörschwellenbestimmung

Wenn bei Abarbeitung des oben beschriebenen Algorithmus zur Bestimmung der Hörschwelle bei einem Reizpegel von x dB noch ein AEP nachweisbar ist, beim nächst niedrigeren Reizpegel y dB aber kein AEP nachweisbar ist, besteht eine gewisse Wahrscheinlichkeit bei dem nachgewiesenen AEP für ein falsch-positives Ergebnis. Deshalb wird vorschlagsgemäß die Untersuchung beim Pegel x wiederholt. Wird bei der Wiederholung kein AEP nachgewiesen, war das vorherige Ergebnis beim Pegel x mit sehr hoher Wahrscheinlichkeit falsch-positiv, und es muß bei einem erhöhten Reizpegel erneut getestet werden. Wird dagegen bei der Wiederholungsmessung mit Reizpegel x erneut ein AEP nachgewiesen, so kann mit sehr hoher Wahrscheinlichkeit davon ausgegangen werden, daß ein AEP vorliegt, da ein falsch-positives Ergebnis nur zufällig auftritt und sich nicht reproduziert. Dieses Vorgehen bewirkt zwar eine Verlängerung der Untersuchungsdauer, die bei dieser Untersuchung aber toleriert werden kann.

2. Hörscreening

Bei einem Hörscreening ist eine kurze Untersuchungsdauer erforderlich. Das für die Hörschwellenbestimmung beschriebene Vorgehen ist deshalb hier nicht zu empfehlen. Untersuchungen haben gezeigt, daß es günstig ist, parallel zur Testung im Spektralbereich einen im Zeitbereich arbeitenden statistischen Test einzusetzen. Da im Zeit- und Spektralbereich nicht identische Merkmale ausgewertet werden (im Spektralbereich vorrangig das Phasenspektrum, im Zeitbereich die Amplituden der Zeitfunktion), wird ein falsch-positives Ergebnis des Tests im Spektralbereich häufig nicht durch ein gleiches Ergebnis im Zeitbereich bestätigt und umgekehrt. Deshalb kann durch eine UND-Verknüpfung der Testergebnisse im Zeit- und Spektralbereich die Anzahl falsch-positiver Ergebnisse erheblich reduziert werden. Als Test im Zeitbereich wird der Friedman Test (L. Sachs, Angewandte Statistik, Springer Verlag 1992, 7. Auflage) eingesetzt. Die AEP-Nachweisempfindlichkeit des Friedman Testes ist allerdings geringer als die Sensitivität des modifizierten q-sample uniform scores Testes. Infolge der UND-Verknüpfung resultiert daraus eine insgesamt geringere Nachweisempfindlichkeit. Da beim Hörscreening aber mit einem für das Normalgehör leicht überschwelligen Reizpegel (15 oder 20 dB) gearbeitet wird, bei dem auch der Friedman Test eine hundertprozentige AEP-Nachweisrate aufweist, ist die Reduzierung der Testempfindlichkeit bei dieser Anwendung unkritisch.

Bei der Hörschwellenbestimmung kann durch die für das Hörscreening beschriebene Kombination der Testung im Zeit- und Spektralbereich eine Erhöhung der AEP-Nachweisempfindlichkeit erzielt werden, wenn statt der UND-Verknüpfung eine ODER-Verknüpfung der Testergebnisse gewählt wird. Einer dadurch bewirkten Erhöhung der Anzahl falsch-positiver Ergebnisse kann durch den oben beschriebenen Wiederholungsalgorithmus entgegengewirkt werden.

Die Reizwiederholrate ist mit 59/s für FAEP-Ableitungen ungewöhnlich hoch gewählt, d.h. es werden 59 Sweeps pro Sekunde gewonnen (37 Sweeps pro Sekunde bei dem im Stand der Technik genannten Vergleichsverfahren). Jede andere Reizwiederholrate zwischen etwa 10/s und etwa 100/s ist prinzipiell denkbar.

Es ist bekannt, daß sich die Amplitude der Welle V der FAEP bei Erhöhung der Reizwiederholrate verringert. Bis zu einer Reizwiederholrate von etwa 100/s ist die Reduzierung der Amplitude aber nur relativ gering. Jede Verringerung der Amplitude verringert zwar das Signal-Rausch-Verhältnis und damit die Nachweismöglichkeit, bei der erhöhten Reizrate werden dafür aber mehr Sweeps pro Zeiteinheit gemittelt. Die dadurch erzielte Vergrößerung des Signal-Rausch-Verhältnisses übersteigt die Wirkung der durch die erhöhte Reizwiederholrate bedingten Amplitudenminderung. In der Summe ergibt sich deshalb bei der hohen Reizrate eine bessere AEP-Nachweisbarkeit.

Die Ableitung von 12.000 Sweeps dauert nur etwas mehr als 3 min.

Da der Nachweis von überschwelligen Antworten erheblich weniger Sweeps und damit weniger Zeit kostet und die Schwelleneingrenzung die Anwendung von nur wenigen Reizpegeln erfordert, ist eine komplette Click-Hörschwellenbestimmung im Mittel bei einem Zeitaufwand von weniger als 10 min realisierbar.

Die Anwendung der Erfindung ist mit folgenden Vorteilen verbunden.

Infolge der höheren Potential-Nachweisempfindlichkeit des gewählten und modifizierten statistischen Verfahrens wird bei Anwendung von AEP ein Potentialnachweis in Schwellennähe und damit eine echte Hörschwellenbestimmung möglich. Dadurch wird wesentlich mehr diagnostische Information gewonnen als bei der einfachen ja/nein-Entscheidung des im Stand der Technik genannten Screening-Gerätes.

Der erforderliche geringe Zeitaufwand erlaubt trotzdem einen Einsatz des Verfahrens wie ein Screening-Verfahren. Bei der Realisierung ist auch die Wahl zwischen zwei Betriebsarten denkbar:

1. Automatisches Screening mit einem festen Reizschallpegel von z.B. 25 dB HL
2. Automatische Hörschwellenbestimmung

Weiterhin gibt es keinerlei Voraussetzung bzw. Einschränkung zur Form des Antwortpotentials. Das ist besonders wichtig nicht nur wegen der interindividuellen Variation des FAEP-Antwortmusters, sondern auch aus folgendem weiteren Grund: Bei den (aus Zeitgründen notwendigen) höheren Reizwiederholraten kommt es zu einer überlagerung des durch den aktuellen Reiz evozierten FAEP mit mittellatenten (späteren) Komponenten der durch den vorhergehenden Reiz evozierten Antwort. Bei Säuglingen ist das zwar kaum der Fall, da die mittellatenten Komponenten noch wenig ausgeprägt sind. Mit zunehmendem Alter der untersuchten Kinder kommt es jedoch als Folge der Überlagerung zunehmend zu Formveranderungen der Antwort, die wegen der interindividuell relativ stark streuenden Latenzen der mittellatenten Komponenten variieren.

Bei einem mit einem Template arbeitenden Verfahren ist deshalb bei der Untersuchung älterer Kinder eine verringerte Nachweisempfindlichkeit wahrscheinlich. Als Folge davon wird der Altersbereich, bei dem ein solches Verfahren anwendbar ist, eingeschränkt.

Bei dem hier vorgeschlagenen Verfahren ist dagegen die mittellatente überlagerung nicht nur nicht kritisch, sondern sogar erwünscht, da sie die Nachweisempfindlichkeit erhöht. Es gibt deshalb auch keine altersabhängige Anwendungsbeschränkung.

Die hohe Reizwiederholrate von 59/s (Vergleichsverfahren 37/s) erlaubt eine kurze Untersuchungsdauer.

Das erfindungsgemäße statistische Verfahren ist ein q-sample Test, er liefert, unabhängig davon, wieviel Spektralterme in den Test einbezogen werden, immer nur eine einzige statistische Aussage.

Das erfindungsgemäße Verfahren ist auch auf alle anderen bekannten AEP-Arten und OAE-Emissionen anwendbar. Es kann unmittelbar (TEOAE) bzw. nach geringfügiger Modifikation (DPOAE) zum objektiven Nachweis von otoakustischen Emissionen eingesetzt werden.

Es ist darüberhinaus nicht nur anwendbar auf die clickevozierten FAEP, sondern kann auch zur Hörschwellenbestimmung mittels frequenzspezifischer FAEP sowie der Hörschwellenbestimmung mittels aller anderen bekannten AEP-Arten (z.B. mittellatente AEP (MAEP) und kortikale AEP (CAEP)) eingesetzt werden.

Vorteile bei der Anwendung des neuen statistischen Verfahrens auf otoakustische Emissionen (OAE) sind die Verkürzung der Untersuchungsdauer und die größere Sicherheit bei der Entscheidung OAE vorhanden/nicht vorhanden.

Das erfindungsgemäße Verfahren wird nachfolgend an einem Anwendungsbeispiel erläutert.

Bei einem Säugling, bei dem Verdacht auf eine Hörstörung besteht, soll, da die übliche audiometrische Bestimmung der frequenzabhängigen Hörschwelle in diesem Alter nicht möglich ist, objektiv die Click-Hörschwelle ermittelt werden.

Dazu sollen die frühen akustisch evozierten Potentiale (FAEP) bei Click-Stimulation abgeleitet und durch ein statistisches Verfahren nachgewiesen werden.

Die Untersuchung erfolgt während des natürlichen Schlafs des Kindes oder gegebenenfalls unter leichter Sedierung.

Die Reizapplikation erfolgt über Kopfhörer bzw. über Einsteckhörer. Der Startreizpegel für die objektive Hörschwellenbestimmung nach der hier vorgeschlagenen Methode beträgt 20 dB HL. Zur Ableitung des EEG werden 3 Klebe-Elektroden auf die Kopfhaut des Kindes fixiert (Vertex, ipsilaterales Mastoid. Stirn). Die Click-Stimulation erfolgt mit einer Reizwiederholrate von 59/s.

Das EEG wird geeignet verstärkt und anschließend über einen Analog/Digital-Wandler (ADC) in eine digitale Form überführt. Die Abtastrate des ADC beträgt 10 kHz. Vom Reizbeginn zählend, werden jeweils EEG-Abschnitte (Sweeps) einer Länge von 16 ms erfaßt. Jeder Sweep besteht damit aus 160 Abtastwerten. Von jeweils 100 aufeinanderfolgenden Sweeps wird der Mittelwert berechnet. Das resultierende Teilmittelwertpotential wird gespeichert.

Von einem digitalen Signalprozessor mit Parallelverarbeitungseigenschaft wird, während das nächste TMP gemittelt wird, das Frequenzspektrum des bereits gespeicherten TMP berechnet. Dazu werden die ersten 10 Abtastwerte (entsprechend einer Millisekunde) wegen einer möglichen Überlagerung durch einen Reizartefakt verworfen. Das so reduzierte TMP wird am Anfang und Ende cos-getapert und dann mit Null-Werten ergänzt, so daß sich eine Gesamtanzahl von 1024 Abtastwerten ergibt. Anschließend erfolgt eine Fast Fourier Transformation (FFT). Von dem als Ergebnis vorliegenden Spektrum, bestehend aus einem Amplitudenund einem Phasenspektrum, werden nur die ersten $n = 65$ Frequenzterme weiterverarbeitet. Da die Frequenzauflösung bei den gewählten Parametern etwa 10 Hz beträgt, überdecken die 65 Terme den Frequenzbereich bis etwa 650 Hz. Um mögliche Einstreuungen der Netzfrequenz auf das abgeleitete EEG auszublenden, werden die ersten 5 Frequenzterme von diesen 65 Termen nicht in die Auswertung einbezogen.

Sobald die Spektren der ersten $m = 5$ TMP vorliegen, wird parallel zur weiterlaufenden Datenerfassung und TMP-Mittelung automatisch die statistische Testung gestartet. Der erfindungsgemäß modifizierte q-sample-uniform scores Test wird zunächst auf die Stichprobe, bestehend aus den Spektraltermen 6 bis 10 der ersten 5 Teilmittelwertpotentiale angewendet.

Ist das Testergebnis des ersten Tests bei 20 dB nicht signifikant, wird die Anzahl n der einbezogenen Spektralterme schrittweise erhöht, bis sich ein signifikantes Ergebnis ergibt oder bis bei der schrittweisen Erhöhung der Anzahl n der Spektralterme auch der 65. Terme einbezogen worden ist.

Im letzteren Fall wird die statistische Testung bei dem Startreizpegel von 20 dB fortgesetzt, sobald die Spektren der nächsten 5 TMP vorliegen. Die nun erweiterte Stichprobe besteht aus den Spektren der ersten 10 TMP. Hier werden ebenfalls, beginnend bei dem 6. Term, zunächst die ersten $q_1 = 5$ Terme einbezogen, dann wird die Anzahl der einbezogenen Terme schrittweise um jeweils 5 bis zum Erreichen des 65. Terms erhöht, falls sich nicht vorher Signifikanz ergibt.

Sind auch bei der Testung der ersten $m = 10$ TMP alle Testergebnisse nicht signifikant, werden die nächsten 5 TMP mit einbezogen und die Testung läuft wieder wie beschrieben ab.

Es sei hier angenommen, daß sich beim Testen der ersten $m = 20$ TMP ein signifikantes Testergebnis bei $n = 35$ einbezogenen Spektraltermen ergibt. Da mit einem Signifikanzniveau von 0,05 % getestet wird, kann mit einer sehr hohen Wahrscheinlichkeit angenommen werden, daß ein FAEP vorhanden ist. Die Datengewinnung bei dem Reizpegel 20 dB HL wird automatisch abgebrochen und mit 10 dB HL erneut automatisch gestartet. Sobald die ersten $m = 5$ TMP bei dem neuen Reizpegel vorliegen, wird der oben beschriebene Testalgorithmus erneut gestartet.

Es sei angenommen, daß sich hier erst beim Testen der ersten $m = 70$ TMP ein signifikantes Ergebnis bei $n = 45$ einbezogenen Spektraltermen ergibt. Deshalb wird zu diesem Zeitpunkt die Datengewinnung beim Reizpegel 10 dB HL automatisch abgebrochen und mit einem Reipegel von 5 dB HL neu gestartet.

Es sei angenommen, daß auch bei einem Stichprobenumfang von $m = 120$ TMP auch bei Einbeziehung aller $n = 60$ Terme (6. - 65. Term) kein signifikantes Ergebnis erhalten wird. Die Untersuchung bei dem Reizpegel 5 dB wird dann automatisch abgebrochen mit dem Ergebnis, daß bei einem Reizpegel von 5 dB HL kein FAEP nachweisbar ist.

Die objektive Hörschwellenbestimmung ist damit beendet. Das Ergebnis lautet, daß die Click-Hörschwelle des Säuglings bei 10 dB HL liegt, der Säugling also normalhörend und nicht, wie vermutet, hörgestört ist.

Die Untersuchung bei 5 dB HL wurde nur zur vollständigen Beschreibung des Algorithmus angeführt. In der Praxis steht die Diagnose "Normalgehör" bereits bei einer Click-Hörschwelle von 10 dB HL fest. Die Untersuchung bei 5 dB HL würde also nicht gestartet.

Der Zeitbedarf für das automatische Auffinden der Schwelle wäre im obigen Fall etwas weniger als 3 Minuten (der Zeitbedarf für die Gewinnung eines TMP beträgt 1,69 Sekunden; $1,69 \cdot (20+70)$ TMP $\approx 2,5$ Minuten).

Wäre beim Reizpegel von 20 dB HL auch mit einem Stichprobenumfang von $m = 120$ TMP kein Potential nachweisbar gewesen, so wäre der Reizpegel automatisch auf 40 dB HL erhöht worden. Hätte sich auch hier keine Signifikanz ergeben, wäre eine automatische Erhöhung von 80 dB HL erfolgt. Angenommen, daß bei diesem Reizpegel ein FAEP nachweisbar ist, wird anschließend automatisch auf 60 dB erniedrigt und erneut getestet. Wird bei diesem Pegel kein positives Ergebnis erhalten, wird automatisch auf 70 dB HL erhöht. Wird hier ein FAEP nachgewiesen, wird die Untersuchung beendet mit dem Ergebnis, daß die Click-Hörschwelle bei 70 dB HL liegt und der Säugling, wie vermutet, auf dem untersuchten Ohr eine erhebliche Hörminderung hat.

Das Ergebnis der Untersuchung wird zusammen mit den Patientendaten übersichtlich ausgedruckt. Optional werden auch die gemittelten FAEP (Mittelwert der jeweiligen TMP) unter Angabe der jeweiligen Reizparameter mit ausgedruckt.

**Patentansprüche**

1. Verfahren zur automatischen Bestimmung des Hörvermögens, insbesondere bei Neugeborenen und Kleinkindern, das von der Ermittelung der frühen akustisch evozierten Potentiale FAEP oder von otoakustischen Emissionen, DAE ausgeht, bei dem die Testung im Spektralbereich mit den folgenden Schritten durchgeführt wird

   a) es erfolgt eine Vormittelung einer ausreichenden Anzahl von Sweeps zu Teilmittelwertpotentialen TMP, wobei Sweeps als post-stimulus-Abschnitte des Spontan-EEG definiert sind,

   b) von diesen Teilmittelwertpotentialen werden die Spektren berechnet,

   c) auf diese Spektren der Teilmittelwertpotentiale werden der q-sample uniform scores Test in herkömmlicher Weise oder der q-sample Watson $U^2$ Test angewendet, d.h. es werden die Phasen der einbezogenen Spektralterme auf Gleichheit geprüft, oder es wird ein modifizierterq-sample uniform scores Test angewendet, der zusätzlich auch die spektralen Amplituden einbezieht,

   d) die Aussage, welche und wieviel Spektralterme n in den Test einbezogen werden sollen, ergibt sich aus den folgenden Verfahrensschritten

   d1) aus einer ersten Anzahl von Sweeps werden fortlaufend m Teilmittelwertpotentiale gemittelt; auf diese Stichprobe, nach Transformation in den Spektralbereich, bestehend aus m Spektren der Teilmittelwertpotentiale, werden der q-sample uniform scores Test in herkömmlicher oder modifizierter Form oder der q-sample Watson $U^2$ Test mehrfach angewendet;

   d2) vorzugsweise beginnend mit dem ersten Term oberhalb 50 Hz erfolgt ein Testlauf mit dem q-sample uniform scores Test bei Einbeziehung einer geringen Anzahl $q_1$ von Termen;

   d3) bei schrittweiser Erhöhung der Anzahl der in die Testung einbezogenen Terme erfolgt jeweils ein erneuter Testlauf, bis alle n Spektralterme eingezogen wurden, falls nicht vorher das Vorhandensein eines FAEP angezeigt wird;

   d4) hat sich bei der Stichprobe im Umfang von m Teilmittelwertpotentialen auch bei Einbeziehung aller n Spektralterme keine Signifikanz ergeben, so wird bei Vorliegen der nächsten zweckmäßigerweise 5 Teilmittelwertpotentiale der beschriebene Verfahrensablauf auf die vergrößerte Stichprobe mit dem Umfang m + 5 angewendet; eine weitere Vergrößerung des Stichprobenumfangs auch in kleineren oder größeren Schritten mit parallel laufender Testung ist möglich;

   d5) nach Erreichen eines vorgegebenen Stichprobenumfangs im Bereich von etwa m = 50 ... 200 wird, falls der Test keine Signifikanz anzeigt, die weitere Datengewinnung bei diesem Reizpegel abgebrochen oder der Reizpegel wird erhöht;

   d6) wird im Verlauf des beschriebenen Testverfahrens bei einem der Tests das Testergebnis "FAEP vorhanden" angezeigt, so wird die Testung bei diesem akustischen Reizpegel abgebrochen oder nachfolgend der Reizpegel in Schritten von mindestens 5 dB verringert, und es werden erneut die beschriebenen Testläufe, beginnend mit einem Stichprobenumfang von m, gestartet.

2. Verfahren nach Anspruch 1,
   **dadurch gekennzeichnet, daß** die in den Prozeß der Vormittelung zu einem Teilmittelwertpotential (TMP) einbezogene Anzahl von Sweeps 50 ... 1000 beträgt, wobei die Anzahl der Sweeps pro TMP von der parallel berechneten Varianz der TMP Sinalrauschverhältnis abhängig gemacht werden kann.

3. Verfahren nach Anspruch 2,
   **dadurch gekennzeichnet, daß** vorzugsweise 100 Sweeps pro Teilmittelwertpotential gemittelt werden.

4. Verfahren nach einem der Ansprüche 1 bis 3,
   **dadurch gekennzeichnet, daß** das Verfahren gestartet wird bei einer Zahl m der gemittelten Teilmittelwertpotentiale von $m \geq 3$, vorzugsweise m = 5, oder m = 10.

5. Verfahren nach Anspruch 1,
**dadurch gekennzeichnet, daß** die Mehrfachtestung nach dem Verfahrenschritt d) mit $q_1 \geq 2$ Spektraltermen beginnt.

6. Verfahren nach einem der Ansprüche 1 bis 5,
**dadurch gekennzeichnet, daß** bei der schrittweisen Erhöhung der Anzahl n der getesteten Spektralterme stets eine bestimmte, ebenfalls schrittweise erhöhbare Anzahl von Spektraltermen hinzugefügt wird, die durch Simulation erzeugt worden sind und nur zufallsverteilte Phasen und Amplituden enthalten.

7. Verfahren nach einem der Ansprüche 1 bis 6,
**dadurch gekennzeichnet, daß** die Reizwiederholrate größer als 10/s ist und vorzugsweise 59/s betragen soll.

8. Verfahren nach einem der Ansprüche 1 bis 7,
**dadurch gekennzeichnet, daß** für die Schritte bei der Verringerung bzw. Erhöhung des Reizpegels ein Algorithmus gewählt wird, dessen Prinzip darin besteht, daß ein vorgegebener Testspielraum für den Reizpegel beim folgenden Test halbiert wird; ist dagegen bei einer notwendigen Reizpegelerhöhung der Testspielraum nach oben noch offen, erfolgt für den nächsten Test eine Verdopplung des aktuellen Reizpegels.

9. Verfahren nach einem der Ansprüche 1 bis 8,
**dadurch gekennzeichne**t, daß für die Anwendung des modifizierten q-sample uniform scores Test gilt mit

- n = Anzahl der einbezogenen Spektralterme,
- m = Anzahl der Teilmittelwertpotentiale (TMP),
- es liegen h = m - n Phasenwinkel vor,
- alle h Phasenwinkel werden in eine Rangordnung gebracht, Bindungen randomisiert aufbrechen, $r_{ik}$ sind die Ränge der Phasen im i-ten Spektralterm (i = 1, ... ,n) des k-ten TMP (k = 1, ... ,m)
- es werden alle Phasen ersetzt durch

$$\beta_{ik} = \frac{2 \cdot \Pi \cdot r_{ik}}{h}$$

mit der Modifikation:

- es werden die spektralen Amplituden $A_{ik}$ für jedes TMP getrennt in eine Rangfolge gebracht, oder es werden alle h = m - n spektralen Amplituden insgesamt in eine Rangfolge gebracht, wobei $a_{ik}$ die Ränge der Amplituden $A_{ik}$ im i-ten Spektralterm des k-ten TMP sind;
  Teststatistik:

$$w^* = \frac{4}{n^2 \cdot (n+1)^2} \cdot \frac{120}{m} \cdot \sum_{i=1}^{n} (C_i^{*2} + S_i^{*2})$$

mit

$$C_i^* = \sum_{k=1}^{m} a_{ik} \cdot \cos \beta_{ik}; \quad S_i^* = \sum_{k=1}^{m} a_{ik} \cdot \sin \beta_{ik}$$

10. Verfahren nach einem der Ansprüche 1 bis 9,
**dadurch gekennzeichnet, daß** es mit transitorisch evozierten OAE (TEOAE) oder mittels Distorsionsprodukten otoakustischer Emissionen (DPOAE) zum objektiven Nachweis von otoakustischen Emissionen eingesetzt wird.

11. Verfahren nach einem der Ansprüche 1 bis 10,

# EP 0 869 737 B1

**dadurch gekennzeichnet, daß** für die Durchführung eines Hörscreenings parallel zur Testung im Spektralbereich ein im Zeitbereich arbeitender statistischer Test eingesetzt wird, wobei im Spektralbereich vorrangig das Phasenspektrum und im Zeitbereich die Amplituden der Zeitfunktion ausgewertet werden und mittels einer UND-Verknüpfung die Testergebnisse im Zeit- und Spektralbereich kombiniert ausgewertet werden, so daß ein falsch-positives Ergebnis aus dem Test im Spektralbereich häufig nicht durch ein gleiches Ergebnis im Zeitbereich bestätigt wird und umgekehrt.

12. Verfahren nach Anspruch 11,
**dadurch gekennzeichnet, daß** zur Erhöhung der AEP-Nachweisempfindlichkeit bei der Hörschwellenbestimmung mittels der für die Durchführung des Hörscreenings angewendeten Kombination der Testung im Zeit- und Spektralbereich anstelle einer UND-Verknüpfung eine ODER-Verknüpfung der Testergebnisse erfolgt.

**Claims**

1. Process for the automatic determination of hearing, particularly of newborns and infants, which is based on the determination of the auditory brainstem responses, ABRs, or otoacoustic emissions, OAEs, in which the testing in the frequency domain is carried out with the following steps

> a) a pre-averaging of a sufficient number of sweeps yielding subaverages, SA, is done wherein sweeps are defined as post-stimulus segments,
> b) the spectra are calculated from these subaverages,
> c) the q-sample uniform scores test in conventional manner or the q-sample Watson $U^2$ test are applied to these spectra of the subaverages, i.e., the phases of the included spectral terms are checked for equality, or a modified q-sample uniform scores test is applied, which, in addition, the spectral amplitudes also includes,
> d) the statement which and how many spectral terms n are to be included in the test, results from the following steps of the process

>> d1) continuously m subaverages are averaged based on a first number of sweeps; the q-sample uniform scores test in conventional manner or the q-sample Watson $U^2$ test are repeatedly applied to this sample, after transformation into the frequency domain, consisting of m spectra of the subaverages;
>> d2) preferably starting with the first term above 50 Hz a test run is carried out using the q-sample uniform scores test including a small number $q_1$ of terms;
>> d3) increasing the number of the included terms step by step another test run is carried out at each step until all n spectral terms have been included unless an ABR has been indicated before;
>> d4) if with the sample size of m subaverages even including all n spectral terms no significance has shown, the process mentioned is, when the next practically 5 subaverages are available, applied to the extended sample of the size m + 5; a further extension of the sample, also in smaller or larger steps with the testing running parallel is possible;
>> d5) after having reached a predetermined sample size ranging from about m = 50 to 200, unless the test does not show significance, the further data acquisition is broken off at this stimulation level or the stimulation level is increased;
>> d6) if in the course of the test process described the test result "ABR present" is indicated in one of the tests, the testing is stopped at this acoustic stimulation level or subsequently the stimulation level is decreased in steps of at least 5 dB, and the test runs described are restarted beginning with a sample size of m.

2. Process of Claim 1,
wherein the number of sweeps included in the process of pre-averaging to give a subaverages (SA) is 50-1000, whereby the number of sweeps per SA can be chosen dependent upon the variance of the SA, signal-to-noise ratio, calculated in parallel.

3. Process of Claim 2,
wherein preferably 100 sweeps per subaverage are averaged.

4. Process of any of the Claims 1 to 3,
wherein the process is started at a number m of averaged subaverages with $m \geq 3$, preferably m = 5, or m = 10.

**5.** Process of Claim 1,
wherein the repeated testing starts after process step d) at $q_i \geq 2$ spectral terms.

**6.** Process of any of the Claims 1 to 5,
wherein during the stepwise increase of the number n of tested spectral terms always a defined, also stepwisely increasable number of spectral terms is added, which have been produced by simulation and contain only randomly distributed phases and amplitudes.

**7.** Process of any of the Claims 1 to 6,
wherein the stimulation repetition rate is greater than 10/s and preferably is to be 59/s.

**8.** Process of any of the Claims 1 to 7,
wherein for the steps of increasing or decreasing the stimulation level, an algorithm is chosen the principle of which is that a predetermined stimulation level range is cut in half at the following test; if, on the other hand, the stimulation level range is upwards open yet on a necessary increase of the stimulation level, the appropriate stimulation level is doubled for the next test.

**9.** Process of any of the Claims 1 to 8,
**characterized by** the fact that when the modified q-sample uniform scores test is employed it applies with

- n = number of the spectral terms included,
- m = number of the subaverages (SAs),
- there are $h = m \times n$ phase angles,
- all h phase angles are ranked, links randomizedly broken up, $r_{ik}$ are the ranks of the phases in the i-th spectral term (i = 1, ..., n) of the k-th SA (k = 1, ..., m)
- all phases are substituted by

$$\beta_{ik} = \frac{2 \cdot \Pi \cdot r_{ik}}{h}$$

with the modification:
- the spectral amplitudes $A_{ik}$ of each SA are separately ranked, or all $h = m \times n$ spectral amplitudes are ranked altogether whereby $a_{ik}$ are the ranks of the amplitudes $A_{ik}$ in the i-th spectral term of the k-th SA;
Test statistics

$$w^{\bullet} = \frac{4}{n^2 \cdot (n+1)^2} \cdot \frac{120}{m} \cdot \sum_{i=1}^{n} (C_i^{\bullet 2} + S_i^{\bullet 2})$$

*mit*

$$C_i^{\bullet} = \sum_{k=1}^{m} a_{ik} \cdot \cos \beta_{ik}; \quad S_i^{\bullet} = \sum_{k=1}^{m} a_{ik} \cdot \sin \beta_{ik}$$

**10.** Process of any of the Claims 1 to 9,
**characterized by** the fact that it is applied with transitorily evoked OAE (TEOAE) or by means of distortion product otoacoustic emissions (DPOAE) to detect otoacoustic emissions objectively.

**11.** Process of any of the Claims 1 to 10,
wherein for performing a hearing screening a statistical test operating in the time domain is employed parallel to the testing in the frequency domain with the phase spectrum predominantly evaluated in the frequency domain and the amplitudes of the time function in the time domain and by means of an AND-operation the test results in the time and frequency domains are evaluated in combination so that an false-positive test result of the test in the frequency domain is frequently not affirmed in the time domain and vice versa.

12. Process of Claim 11,
wherein for increasing the AEP detection sensitivity during the hearing threshold determination through the combination of testing in the time and frequency domains, which is used for performing the hearing screening, instead of an AND-operation an OR-operation of the test results is made.

**Revendications**

1. Procédé de contrôle automatique de l'acuité auditive, notamment de nouveau-nés et de nourrissons qui se base sur la détermination du potentiel prématuré évoqué acoustiquement ou sur des émissions oto-acoustiques, et qui prévoit des contrôles dans le domaine spectral en étapes comme suit :

a) préalablement, on établit les moyennes d'un nombre suffisant de sweeps qui donneront le potentiel en moyennes partielles (p.m.p.), les sweeps y sont définis comme sections après-stimulation de l'électro-encéphalogramme spontané,

b) avec les valeurs du potentiel en moyennes partielles, on calcule les spectres,

c) sur ces spectres du potentiel en moyennes partielles, on applique le test q-sample uniform scores de la manière traditionnelle ou le test q-sample Watson $U^2$, c'est-à-dire, on vérifie la similitude des phases des valeurs intégrées du spectre, ou on applique un test q-sample uniform scores modifié, qui comprend encore les amplitudes spectrales,

d) afin de décider combien de valeurs du spectre n et lesquelles doivent être intégrées dans ce test, les étapes suivantes du procédé sont nécessaires :

d 1) à partir d'un premier nombre de sweeps, on détermine en continu le potentiel en moyennes partielles ; sur cette épreuve au hasard, après la transformation dans le domaine spectral qui est composé de m spectres du potentiel en moyennes partielles, on applique plusieurs fois le test q-sample uniform de la manière traditionnelle ou modifiée, ou bien on y applique plusieurs fois le test q-sample Watson $U^2$;

d 2) commençant de préférence par la première valeur supérieure à 50 Hz, on fait un premier programme de contrôle avec le test q-sample uniform, dans lequel on n'emploie qu'un nombre réduit $q_1$ de valeurs ;

d 3) en augmentant pas à pas le nombre de valeurs intégrées dans le test, on effectue à chaque fois un nouveau programme de contrôle, jusqu'à ce que toutes les valeurs du spectre soient tenues en compte, à condition que le dispositif n'indique pas plus tôt l'existence d'un potentiel prématuré évoqué acoustiquement ;

d 4) si l'épreuve au hasard avec m valeurs du potentiel en moyennes partielles n'a donné aucune signification même en y ayant considéré toutes les n valeurs du spectre, on applique le procédé expliqué ci-dessus sur un contrôle au hasard élargi avec m + 5 dès qu'il y a au moins cinq valeurs suivantes du potentiel en moyennes partielles ; il est également possible d'élargir la gamme des épreuves au hasard en plus petits ou plus grands pas en continuant les contrôles en même temps ;

d 5) après l'obtention d'un nombre d'épreuves au hasard prédéfini qui devrait être compris entre m = 50 et m = 200 environ, la collection de données n'est plus continuée à ce niveau de stimulation, si le test n'indique aucune signification, ou bien on augmente le niveau de stimulation ;

d 6) si, au cours de l'un de ces contrôles, le résultat « potentiel prématuré évoqué acoustiquement existe » est indiqué, le test est arrêté à ce niveau de stimulation acoustique, ou ce dernier est baissé progressivement dans des pas d'au moins 5 dB, et on recommence les programmes de contrôle expliqués ci-dessus en commençant par un nombre d'épreuves au hasard de m.

**2.** Procédé selon la revendication n°. 1,
**caractérisé par le fait que** le nombre de sweeps dont on établit préalablement la moyenne afin d'obtenir le potentiel en moyennes partielles (p.m.p.) est compris entre 50 et 1000, le nombre de sweeps par p.m.p. peut varier en fonction de la variance du p.m.p. (rapport signal-bruit) qui est calculée en même temps.

**3.** Procédé selon la revendication n°. 2,
**caractérisé par le fait qu'** on utilise de préférence 100 sweeps par potentiel en moyennes partielles pour en former la moyenne.

**4.** Procédé selon l'une des revendications 1 à 3,
**caractérisé par le fait que** le procédé est entamé quand le nombre m des valeurs du potentiel en moyennes partielles est égal ou supérieur à 3, de préférence il est m = 5 ou m = 10.

**5.** Procédé selon la revendication n°. 1,
**caractérisé par le fait que** le contrôle multiple après la phase d) du procédé commence avec $q_1 \geq 2$ valeurs du spectre.

**6.** Procédé selon l'une des revendications 1 à 5,
**caractérisé par le fait que**, lors d'une augmentation pas à pas du nombre n des valeurs du spectre, on y ajoute, après les avoir testées, un nombre de valeurs du spectre qui découlent d'une simulation et qui ne disposent que phases et d'amplitudes disposées aléatoirement et dont le nombre peut, lui aussi, être augmenté pas à pas.

**7.** Procédé selon l'une des revendications 1 à 6,
**caractérisé par le fait que** le taux de répétition de la stimulation est supérieur à 10/s, et de préférence il est de 59/s.

**8.** Procédé selon l'une des revendications 1 à 7,
**caractérisé par le fait que** pour réduire ou augmenter le niveau de la stimulation, les pas suivent un algorithme dont le principe réside dans **le fait qu'**une marge de contrôle prédéterminée pour le niveau de la stimulation est divisée par moitié dans le test suivant ; si la marge de contrôle est encore ouverte vers le haut quand une augmentation du niveau de la stimulation est nécessaire, il faut doubler le niveau de la stimulation lors du contrôle suivant.

**9.** Procédé selon l'une des revendications 1 à 8,
**caractérisé par le fait que** pour le test q-sample uniform scores s'applique : avec

- n = nombre des valeurs du spectre intégrées,
- m = nombre des potentiels en moyennes partielles (p.m.p.)
- il existe h = m - n angles de phase,
- tous les h angles de phase sont classés par rang, avec rupture randomisée des liaisons, $r_{ik}$ représentent les rangs des phases dans la i-e valeur du spectre (i = 1, ..., n) du k-e p.m.p. (k = 1,... m)
- toutes les phases sont remplacées par

$$\beta_{ik} = \frac{2 \cdot \Pi \cdot r_{ik}}{h}$$

avec la modification :
- toutes les amplitudes spectrales $A_{ik}$ sont classées par rang séparément pour chaque p.m.p., ou la totalité des h = m - n amplitudes spectrales sont classées par rang, $a_{ik}$ y représente les rangs des amplitudes des $A_{ik}$ dans la i-e valeur du spectre du k-e p.m.p.; statistique du test :

$$w^* = \frac{4}{n^2 \cdot (n+1)^2} \cdot \frac{120}{m} \cdot \sum_{i=1}^{n} (C_i^{*2} + S_i^{*2})$$

*mit*

$$C_i^\bullet = \sum_{k=1}^{m} a_{ik} \cdot \cos \beta_{ik}; \quad S_i^\bullet = \sum_{k=1}^{m} a_{ik} \cdot \sin \beta_{ik}$$

10. Procédé selon l'une des revendications 1 à 9,
    **caractérisé par le fait qu'**il est appliqué avec des émissions oto-acoustiques évoquées transitoirement ou à l'aide de produits de distorsion des émissions oto-acoustiques afin de mettre en évidence objective les émissions oto-acoustiques.

11. Procédé selon l'une des revendications 1 à 10,
    **caractérisé par le fait que**, pour contrôler l'acuité auditive avec visualisation sur l'écran, on fait un test dans le domaine spectrale, et en même temps, on en fait un autre, qui est statistique et se fait dans le domaine temporaire ; celui qui se fait dans le domaine spectrale analyse notamment la gamme des phases, et celui qui se fait dans le domaine temporaire analyse les amplitudes de la fonction de temps ; les résultats des tests dans les domaines et de temps et de spectre sont mis en relation et analysés ; ainsi, un faux résultat positif qui résulte du test dans le domaine spectrale n'est pas confirmé par un résultat identique obtenu par le test dans le domaine temporaire - et inversement.

12. Procédé selon la revendication n°. 11,
    **caractérisé par le fait que**, dans le but d'une augmentation de la sensibilité du contrôle du potentiel évoqué acoustiquement lors de la détermination des seuils de acuité auditive, on peut choisir une composition OU à la place d'une composition ET pour mettre en relation les résultats obtenus dans les tests dans le domaine de temps et de spectre, qui font partie du contrôle de l'acuité auditive avec visualisation sur l'écran.